# EUROPÄISCHE PATENTSCHRIFT

(11) **EP 0 596 359 B1**
(45) Veröffentlichungstag und Bekanntmachung des Hinweises auf die Patenterteilung: **03.07.2002**
(21) Anmeldenummer: 93117240.7
(22) Anmeldetag: 25.10.1993
(51) Int. Cl.: C07D 317/46, C07D 317/70, C07D 491/056

(54) **Fluorierte 1,3-Benzo- und 1,3-Pyrido-dioxole, Verfahren zu deren Herstellung und deren Verwendung zur Herstellung von Pflanzenschutzmitteln**
Fluorinated 1,3-benzo- and 1,3-pyrido-dioxoles, process for their preparation and their use for the preparation of plant-protective agents
1,3-benzo- et 1,3-pyrido-dioxoles fluorées, procédé pour leur préparation et leur utilisation pour la préparation des produits protecteurs pour plantes

(30) Priorität: 06.11.1992 DE 4237579
(43) Veröffentlichungstag der Anmeldung: 11.05.1994
(73) Patentinhaber: BAYER AG, 51368 Leverkusen (DE)
(72) Erfinder: Böhm, Stefan, Dr., D-51065 Köln (DE); Marhold, Albrecht, Dr., D-51373 Leverkusen (DE)

(56) Entgegenhaltungen:
- EP-A- 0 011 179
- EP-A- 0 042 533
- EP-A- 0 300 307
- EP-A- 0 596 361
- WO-A-94/11349
- WO-A-94/11350
- WO-A-94/11351
- WO-A-94/11352
- DE-A- 2 624 822
- US-A- 3 948 952
- US-A- 4 838 924
- JOURNAL OF THE INDIAN CHEMICAL SOCIETY Bd. 30, Nr. 12 , 1953 Seiten 809 - 814 HENNE A.L. & LATIF K.A. 'Behaviour of fluorinated olefines towards anionic reagents'
- JOURNAL OF THE CHEMICAL SOCIETY, PERKIN TRANSACTIONS I Nr. 4 , April 1987 , LONDON Seiten 763 - 767 BAYLIFF A.E. ET AL. 'Polyhalogenoheterocyclic compounds. Part 38. Reactions of fluorinated- alkenes and -cycloalkenes with difunctional nucleophiles'
- HELVETICA CHIMICA ACTA Bd. 55, Nr. 7 , 1972 Seiten 2461 - 2467 BERTHOLD R. ET AL. '246. Synthese und Eigenschaften von Spiro[1,3-benzodioxol-2,3'-pyrrolidin]'

## Beschreibung

Die vorliegende Erfindung betrifft neue fluorierte 1,3-Benzodioxole und neue 1,3-Pyridodioxole, deren Herstellung und deren Verwendung als Zwischenprodukte zur Herstellung von Pflanzenschutzmitteln.

Aus der DE-OS 26 24 822 sind 1,3-Benzodioxole bekannt, bei denen das Kohlenstoffatom in 2-Stellung mit 1 bis 2 halogenierten aliphatischen Kohlenwasserstoffresten, z.B. Chlormethyl, substituiert sein kann. Sie werden verwendet, um zunächst das entsprechende 5-Amino-1,3-benzodioxol und daraus ein Harnstoffderivat herzustellen.

In J. Chem. Soc. Perkin Trans. I. 1987, 763 ist die Herstellung von 1,3-Benzodioxolen beschrieben, die in 2-Stellung fluorhaltige, meist cyclische, stets aber ganz oder überwiegend ethylenisch ungesättigte Reste enthalten.

Das Jour. Indian. Chem. Soc. 30, 809 (1953) beschreibt Umsetzungen von Methanol und Ethylenglykol mit fluorierten Olefinen, die geradkettig oder cyclisch sein können. Hier können, je nach Reaktionsbedingungen, gesättigte und ungesättigte Produkte erhalten werden.

Der Stand der Technik liefert keine Hinweise darauf, wie man ausgehend von Benzcatechinen oder Dihydroxypyrimidinen und Hexafluorbutenen in hohen Ausbeuten, Benzo- und Pyrido-Dioxole erhalten kann, die im Dioxolteil gesättigte Substituenten enthalten.

In 2-Stellung CF₃-Gruppen enthaltende 1,3-Benzodioxole und 1,3-Pyridodioxole sind bisher nicht bekannt geworden.

Es wurden nun fluorierte 1,3-Benzo- und 1,3-Pyridodioxole der Formel (I) gefunden 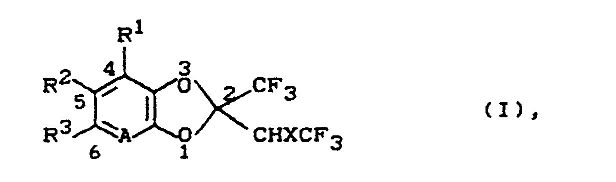 in der
- A: für C-R⁴ oder N und
- X: für Wasserstoff, Fluor, Chlor oder Brom stehen und
- R¹ bis R⁴: gleich oder verschieden voneinander sein können und jeweils Wasserstoff, Halogen, C₁-C₆-Alkyl, C₁-C₆-Alkoxy, Halogen-C₁-C₆-alkyl, C₆-C₁₀-Aryl, CHO, COOH, COCl, CN, OH, NCO, COO-C₁-C₆-Alkyl, NO₂, NH₂, NH-C₁-C₆-Alkyl, N(C₁-C₆-Alkyl)₂, SO₂Cl, SO₃H, SO₃Na oder SO₃K bedeuten,
Vorzugsweise steht in Formel (I) A für C-R⁴ und X für Wasserstoff oder Chlor.

Soweit die Reste R¹ bis R⁴ für Halogen oder Halogen-C₁-C₆-Alkyl stehen bedeutet Halogen beispielsweise Fluor, Chlor oder Brom.

Eine gegebenenfalls substituierte -CH=CH-CH=CH-Brücke kann als Substituenten beispielsweise solche enthalten, wie sie oben für R¹ bis R⁴ angegeben wurden.

Vorzugsweise stehen mindestens zwei der Reste R¹ bis R⁴ für Wasserstoff und der oder die verbleibenden Reste, soweit sie nicht ebenfalls für Wasserstoff stehen, unabhängig voneinander vorzugsweise für Chlor, Brom, Methyl, Ethyl, CH₂Cl, CH₂Br, Phenyl, CHO, COOH, OH, NCO, NO₂, NH₂ und/oder SO₂Cl.

Wenn zwei benachbarte Reste aus der Reihe R¹ bis R⁴ für eine gegebenenfalls substituierte -CH=CH-CH=CH-Brücke stehen, so handelt es sich dabei vorzugsweise um die Reste R² und R³.

Ein bevorzugtes erfindungsgemäßes Verfahren zur Herstellung von 1,3-Benzo- und 1,3-Pyrido-dioxolen der Formel (I) ist dadurch gekennzeichnet, daß man 1,2-Dihydroxybenzole oder 2,3-Dihydroxypyridine der Formel (II) 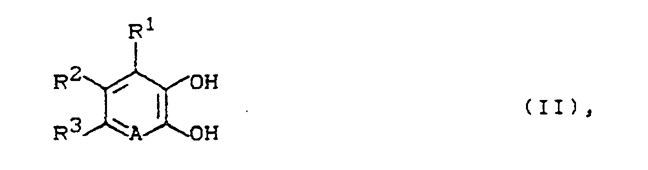 in der
- A und R¹ bis R³: die bei Formel (I) angegebene Bedeutung haben, R¹ bis R³ jedoch nicht für OH, COCl oder SO₂Cl stehen,
in Gegenwart einer Base und eines Verdünnungsmittels bei -20 bis +200°C mit einem Hexafluorbuten der Formel (III) umsetzt 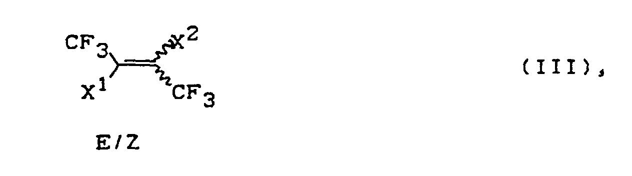 in der
- X¹: für Wasserstoff oder Halogen und
- X²: für Halogen stehen.

Vorzugsweise steht in Formel (III) X¹ für Wasserstoff, Fluor oder Chlor und X² für Fluor oder Chlor.

Die als Ausgangsverbindungen benötigten Verbindungen der Formeln (II) und (III) sind bekannt oder analog bekannten Verbindungen zugänglich. Beispielsweise kann man auf 1 Mol Dihydroxyverbindung der Formel (II) 0,8 bis 1,2 Mole Hexafluorbuten der Formel (III) einsetzen. Vorzugsweise beträgt dieses Verhältnis 1:0,9 bis 1,1, besonders bevorzugt 1:1.

Als Basen kommen praktisch alle gebräuchlichen Basen in Frage, z.B. Natriumhydroxid, Kaliumhydroxid, Natriumcarbonat, Kaliumcarbonat, DBN (= 1,5-Diazabicyclo[4.3.0]-non-5-en), DBU, (= 1,8-Diazabicyclo[4.3.0]-undec-7-en), Pyridin und aliphatische Amine. Organische Basen kommen vorzugsweise in wasserfreier Form zum Einsatz, anorganische können auch als wäßrige Lösung eingesetzt werden. Die Basen können beispielsweise in Mengen von 1 bis 5 Äquivalenten pro Mol Verbindung der Formel (II) eingesetzt werden. Vorzugsweise beträgt diese Menge 1,5 bis 2,5 Äquivalente.

Als Verdünnungsmittel kommen solche in Betracht, die keine aciden Wasserstoffatome enthalten, beispielsweise aliphatische und aromatische Kohlenwasserstoffe, halogenierte Kohlenwasserstoffe und Ether. Bevorzugt sind dipolare, aprotische Lösungsmittel wie Dimethylsulfoxid, N-Methylpyrrolidon, HMPT (= Hexamethylphosphorsäuretriamid), Dimethylformamid und Acetonitril. Verdünnungsmittel werden bevorzugt in Mengen von 100 bis 3000 g pro Mol der Verbindung der Formel (II) eingesetzt.

Dieses erfindungsgemäße Verfahren kann bei erniedrigtem Druck, Normaldruck oder erhöhtem Druck durchgeführt werden. Vorzugsweise arbeitet man bei Normaldruck oder, insbesondere beim Einsatz von niedrigsiedenden Ausgangsmaterialien oder Hilfsmitteln und höheren Reaktionstemperaturen, bei erhöhtem Druck, so daß das Reaktionsgemisch im wesentlich in flüssiger Phase vorliegt.

Bevorzugte Reaktionstemperaturen liegen im Bereich 0 bis 150°C, besonders bevorzugt im Bereich 20 bis 80°C.

Dieses erfindungsgemäße Verfahren wird durch die Beispiele 1 bis 11 illustriert, wobei das Beispiel 2 eine ganz besonders bevorzugte Arbeitsweise beschreibt.

Ein weiteres erfindungsgemäßes Verfahren zur Herstellung von 1,3-Benzo- und 1,3-Pyrido-dioxolen der Formel (I) ist dadurch gekennzeichnet, daß man mit einer Schutzgruppe versehene 1,2-Dihydroxybenzole oder 2,3-Dihydroxypyridine der Formel (IV) 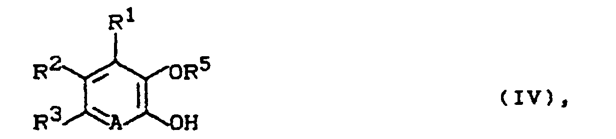 in der
- A und R¹ bis R³: die bei Formel (I) angegebene Bedeutung haben und
- R⁵: für eine Schutzgruppe oder
- R⁵: gemeinsam mit R¹ für einen -C(CH₃)₂-O-Rest stehen,
zunächst mit einem Hexafluorbuten der Formel (III) umsetzt 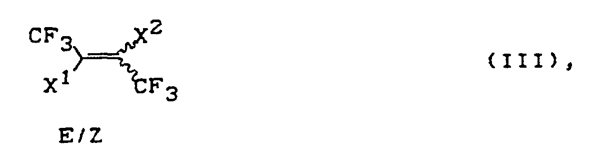 in der
- X¹: für Wasserstoff oder Halogen und
- X²: für Halogen stehen,
so ein Zwischenprodukt der Formel (V) erhält, 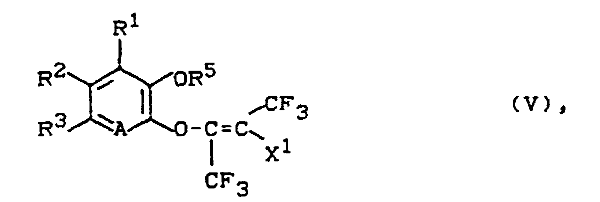 in der
- A und R¹ bis R³: die bei Formel (I),
- R⁵: die bei Formel (IV) und
- X¹: die bei Formel (III)
angegebene Bedeutung haben,
aus dem Zwischenprodukt der Formel (V) die Schutzgruppe R⁵ abspaltet,
die so erhältliche OH-Verbindung mit einer Base umsetzt und so 1,3-Benzo- oder 1,3-Pyrido-dioxole der Formel (I) erhält.

In Formel (IV) steht R⁵ vorzugsweise für C₁-C₄-Alkyl, insbesondere Methyl, für Benzyl oder R⁵ gemeinsam mit R¹ für einen -C(CH₃)₂-O-Rest. Die Verbindungen der Formel (IV) können auf einfache Weise erhalten werden, beispielsweise indem man in Verbindungen der Formel (II) auf an sich bekannte Weise eine Schutzgruppe einführt, beispielsweise durch Umsetzung mit Dimethylsulfat, Benzylchlorid, 2,2-Dimethoxypropan oder ähnlichen Agenzien.

Die Umsetzung einer Verbindung der Formel (IV) mit einem Hexafluorbuten der Formel (III) kann analog der weiter oben beschriebenen Umsetzung einer Verbindung der Formel (II) mit einem Hexafluorbuten der Formel (III) erfolgen. Die bevorzugte Reaktionstemperatur beträgt hier allerdings -10 bis +50°C und die Base wird hier vorzugsweise in Mengen von 1,0 bis 1,2 Äquivalenten pro Mol der Verbindung der Formel (IV) eingesetzt.

Die Abspaltung der Schutzgruppe R⁵ aus dem so erhaltenen Zwischenprodukt der Formel (V) kann beispielsweise erfolgen, indem man etwa im Falle R⁵ = C₁-C₄-Alkyl eine saure Etherspaltung mit Bromwasserstoff durchführt oder im Falle R⁵ = Benzyl eine hydrogenolytische Spaltung.

Die so erhaltene OH-Verbindung wird durch die Einwirkung einer Base zu einem 1,3-Benzo- oder 1,3-Pyrido-dioxol der Formel (I) umgesetzt. Man kann hierbei so verfahren, daß man z.B. die Verbindung der Formel (V), jedoch mit R⁵ = Wasserstoff, gegebenenfalls in einem Lösungsmittel mit einer katalytischen Menge einer Base (analog der weiter oben beschriebenen Arbeitsweise) bei Temperaturen im Bereich -10 bis +100°C umsetzt.

Die Base wird vorzugsweise in einer Menge von 0,01 bis 1 Äquivalenten pro Mol der OH-Verbindung zugesetzt.

Das Herstellungsverfahren für 1,3-Benzo- und 1,3-Pyridodioxolen aus mit Schutzgruppen versehenen 1,2-Dihydroxybenzolen und 2,3-Dihydroxypyridinen der Formel (IV) wird durch die Beispiele 12 bis 23 illustriert.

1,3-Benzo- und 1,3-Pyrido-dioxole der Formel (I), die durch die bisher beschriebenen Verfahren nicht oder nur schwierig erhalten werden können, sind dadurch zugänglich, daß man zunächst nicht oder anders substituierte 1,3-Benzo- oder 1,3-Pyrido-dioxole herstellt und diese modifiziert.

Beispiele für solche Modifizierungen von Verbindungen der Formel (I) sind die Einführung von Nitro- oder Halogengruppen als Substituenten R¹ bis R⁴, vorzugsweise als Substituenten R² und/oder R³, durch elektrophile aromatische Substitution, der Austausch von Halogen- durch Cyano-Gruppen, die Halogenierung von Alkyl- zu Halogenalkyl-Seitenketten und die Hydrierung von NO₂-zu NH₂-Substituenten. Derartige Modifizierungen sind an sich bekannt (siehe z.B. Houben-Weyl, Methoden der organischen Chemie, Band E5, Band V (4), Band XI (1) und weitere).

Eine besondere Modifizierung ist die Hydrierung von 1,3-Benzo- und 1,3-Pyrido-dioxolen der Formel (I) mit X = Chlor, wobei 1,3-Benzo- und 1,3-Pyrido-dioxole mit X = Wasserstoff entstehen. Solche Hydrierungen können z.B. bei 20 bis 200°C, 1 bis 200 bar, in Gegenwart üblicher Lösungsmittel wie Methanol, Tetrahydrofuran oder Dioxan und vorzugsweise in Gegenwart von Basen durchgeführt werden. Die Basen können z.B. in äquimolaren oder höheren Mengen vorliegen, und es kann sich dabei z.B. um Kaliumcarbonat oder Triethylamin handeln. Bei derartigen Hydrierungen können bei R¹, R², R³ und/oder R⁴ = Halogen Kernchloratome mithydriert werden.

Die Modifizierung von 1,3-Benzo- und 1,3-Pyrido-dioxolen wird durch die Beispiele 24 bis 40 illustriert.

Es ist ausgesprochen überraschend, daß mit den erfindungsgemäßen Verfahren 1,2-Dihydroxybenzole und 2,3-Dihydroxypyridine mit Hexafluor-2-butenen sehr selektiv monoalkyliert werden können. Es war zu erwarten, daß in erheblichem Umfang Dialkylierungen und Oligomerisierungen stattfinden.

Fluorierte 1,3-Benzo- und 1,3-Pyrido-dioxole der Formel (I) können als Zwischenprodukte zur Herstellung von biologisch aktiven Verbindungen, beispielsweise Pflanzenschutzmitteln verwendet werden.

Beispielsweise kann man 1,3-Benzo- und 1,3-Pyridodioxole der Formel (I), die eine Aminogruppe enthalten, phosgenieren, z.B. mit Phosgen oder Diphosgen, und so die Aminogruppe in eine Isocyanatogruppe überführen, das so erhaltene Isocyanato-1,3-benzo- oder -1,3-pyridodioxol mit einem Benzimidazol z.B. der Formel (VI) 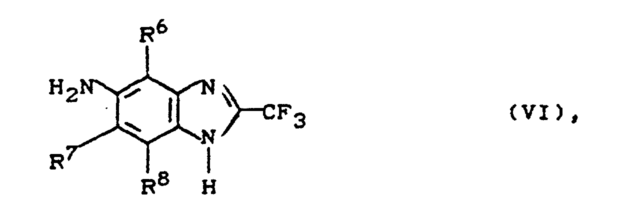 in der
- R⁶, R⁷ und R⁸: unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen, wobei jedoch mindestens einer der Substituenten R⁶, R⁷ oder R⁸ für Halogenalkyl mit Ausnahme des Chlormethylrestes, Halogenalkoxy, Halogenalkylthio, Halogenalkylsulfinyl, Halogenalkylsulfonyl, Alkylsulfonyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Aryl, Arylthio, Arylsulfinyl, Arylsulfonyl, Arylsulfonyloxy, Arylcarbonyl, Aryloxycarbonyl, Arylazo oder Arylthiomethylsulfonyl stehen,
(Herstellung siehe z.B. J. Fluorine Chem. 56, 1 (1992) und dort angegebene Zitate)
umsetzen, so ein Harnstoffderivat z.B. der Formel (VII) erhalten 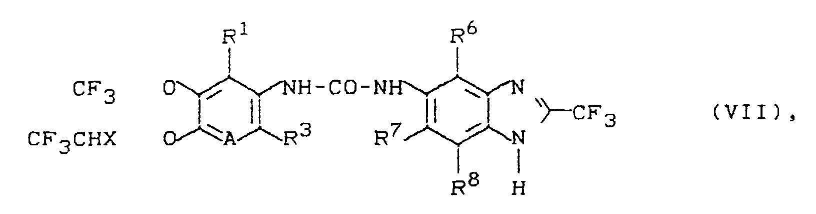 in welcher
R¹, R³, X und A die bei Formel (I) und R⁶, R⁷ und R⁸ die bei Formel (VI) angegebene Bedeutung haben,
und daraus durch Alkylierung mit einem Alkylierungsmittel z.B. der Formel (VIII) 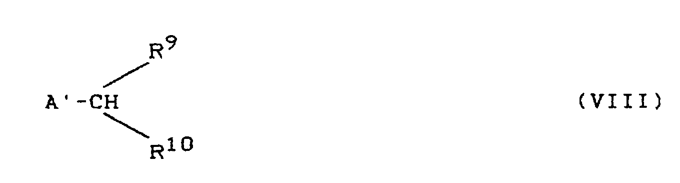 in der
- A': für eine geeignete Abgangsgruppe, z.B. für Halogen, insbesondere für Chlor, Brom oder Iod oder für jeweils gegebenenfalls substituiertes Alkylsulfonyloxy, Alkoxysulfonyloxy oder Arylsulfonyloxy, insbesondere Methansulfonyloxy, Trifluormethansulfonyloxy, methoxysulfonyloxy, Ethoxysulfonyloxy, p-Toluolsulfonyloxy, oder eine Alkohol-, Alkanoyloxy-, Alkoxy- oder Hydroxygruppe, insbesondere eine Acetoxy- oder Methoxygruppe steht,
- R⁹: für Wasserstoff, Alkyl, Alkoxy oder für gegebenenfalls substituiertes Aryl steht und
- R¹⁰: für Hydroxy, Cyano oder für jeweils gegebenenfalls substituiertes Alkyl, Alkenyl, Alkinyl, Alkoxy, Alkenyloxy, Alkinyloxy, Alkylthio, Amino, Aminocarbonyl, Alkylcarbonyl, Alkoxycarbonyl, Alkylcarbonyloxy, Dialkoxyphosphonyl, (Hetero)Aryl, (Hetero)Arylcarbonyl, (Hetero)Aryloxycarbonyl, (Hetero)Arylcarbonyloxy oder (Hetero)Arylaminocarbonylaminocarbonyloxy steht,
ein Harnstoffderivat erhalten, das z.B. der Formel (VII) entspricht, mit dem Unterschied, daß das Stickstoffatom neben R⁸ mit einem 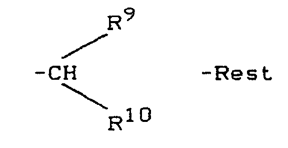 substituiert ist.

Die zur Herstellung solcher Harnstoffderivate nötigen Umsetzungen (Phosgenierung, Harnstoffbildung aus Isocyanat und Amin, Alkylierung) können auf an sich bekannte Weise durchgeführt werden.

Verbindungen der Formel (VIII) sind zum größten Teil bekannt. Nicht bekannt sind beispielsweise Verbindungen der Formel (VIII')

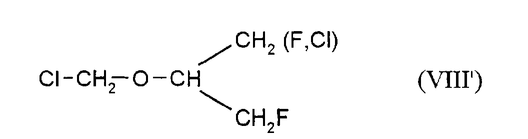

Diese können erhalten werden, indem man das entsprechende Isopropanol mit Formaldehyd und Chlorwasserstoff bei -20 bis +20°C umsetzt. Die Verbindungen der Formel (VIII') und ihre Herstellung sind Gegenstand einer anderen Schutzrechtsanmeldung des gleichen Anmelders (siehe DE-A 4 237 617).

Weiterhin kann man beispielsweise 1,3-Benzo- und 1,3-Pyridodioxole der Formel (I), die zwei benachbarte Aminogruppen enthalten mit Trifluoressigsäure in das entsprechende Benzimidazol z.B. der Formel (IX) überführen 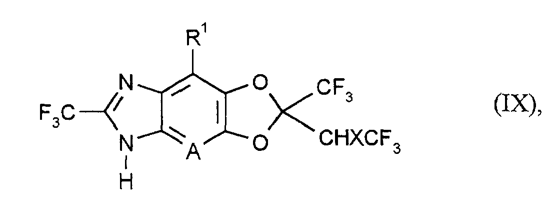 in der
A, R¹ und X die bei Formel (I) angegebene Bedeutung haben.

Aus diesen kann man durch Alkylierung z.B. mit Verbindungen der Formel (VIII) Benzimidazolderivate erhalten, die z.B. der Formel (IX) entsprechen, mit dem Unterschied, daß das Stickstoffatom neben A mit einem 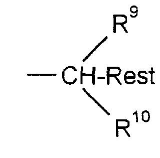 substituiert ist. 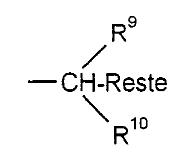 enthaltene, sonst den Formeln (VII) und (IX)
entsprechende Verbindungen sind beispielsweise geeignet zur Bekämpfung von tierischen Schädlingen, wie Arthropoden und Nematoden, insbesondere Insekten und Spinnentieren, die in der Landwirtschaft in Forsten, im Vorrats- und Materialschutz, sowie auf dem Hygienesektor als Schädlinge auftreten. Sie sind gegen normal sensible und resistente Arten, sowie gegen alle oder einzelne Entwicklungsstadien wirksam. Diese Stoffe und deren Herstellung sind Gegenstand einer anderen Schutzrechtsanmeldung des gleichen Anmelders (siehe DE-A 4 237 557).

### Beispiele

### Beispiel 1

### 2-(2,2,2-Trifluorethyl)-2-trifluormethyl-1,3-benzodioxol

11 g Brenzkatechin wurden in 200 ml Dimethylformamid gelöst und mit 18 g 45 gew.-%iger wäßriger Natronlauge versetzt. Die Mischung wurde bei 75°C tropfenweise mit 20 g 2-Chlor-1,1,1,4,4,4-hexafluor-2-buten versetzt. Man rührte 30 Minuten bei 75°C nach. Anschließend wurde der Ansatz auf 500 ml Eiswasser gegossen und mit Diethylether extrahiert. Die organische Phase wurde mit Wasser gewaschen, mit Magnesiumsulfat getrocknet und eingeengt. Das Produkt wurde schließlich im Hochvakuum destilliert. Die Ausbeute betrug 15 g (= 56 %), der Siedepunkt 60°C bei 10 mbar. Die NMR-Spektren zeigten folgende charakteristische Absorptionen: ¹⁹F-NMR: -59,0 und -84,6 ppm. ¹H-NMR: 3,02 ppm.

### Beispiel 2

### 2-(1-Chlor-2,2,2-trifluorethyl)-2-trifluormethyl-1,3-benzodioxol

110 g Brenzkatechin wurden in 1500 ml Acetonitril gelöst und mit 200 g Triethylamin versetzt. Die Mischung wurde bei 75°C tropfenweise mit 235 g 2,3-Dichlor-1,1,1,4,4,4-hexafluor-2-buten versetzt. Man rührte 2 Stunden bei 75°C nach. Anschließend wurden 1200 ml des Lösungsmittels im Vakuum abdestilliert und der Rückstand mit 1500 ml Wasser aufgenommen. Das Produkt wurde mit Diethylether extrahiert, die organische Phase 2 mal mit 10 gew.-%iger wäßriger Natronlauge und 1 mal mit Wasser gewaschen. Nach dem Trocknen mit Magnesiumsulfat wurde eingeengt und im Vakuum fraktioniert destilliert. Die Ausbeute betrug 258 g (= 84 % der Theorie). Der Siedepunkt lag bei 63°C bei 12 mbar. Die NMR-Spektren zeigten folgende charakteristische Absorptionen: ¹⁹F-NMR: -66,8 und -79,7 ppm. ¹H-NMR: 4,71 ppm.

### Beispiele 3 bis 10

Analog zu den Beispielen 1 und 2 wurden die folgenden Beispiele durchgeführt (Einzelheiten siehe Tabelle 1):

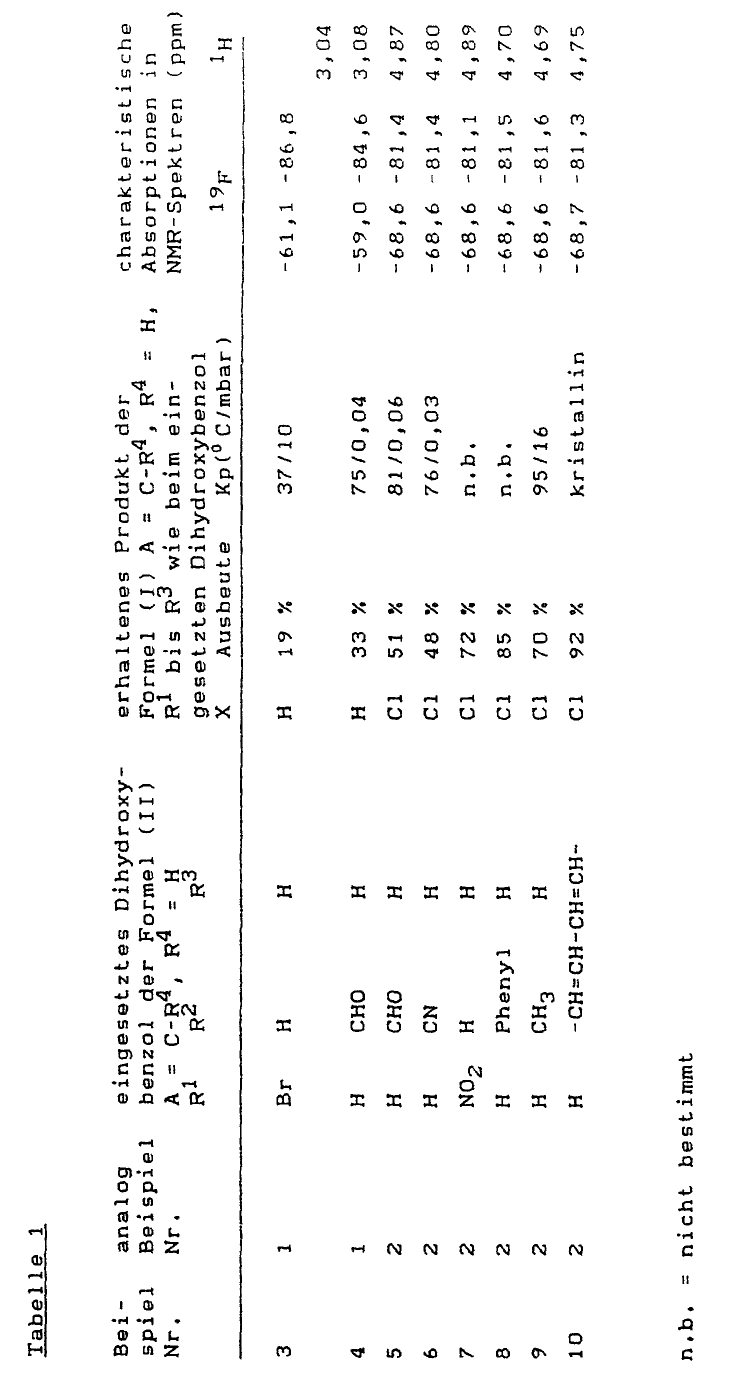

### Beispiel 11

### 2-(1-Chlor-2,2,2-trifluorethyl)-2-trifluormethyl-[1,3]-dioxolo[4,5-b]pyridin

11 g 2,3-Dihydroxypyridin wurden wie in Beispiel 2 beschrieben mit 23,5 g 2,3-Dichlor-1,1,1,4,4,4-hexafluor-2-buten umgesetzt. Nach einer Destillation im Hochvakuum wurde das Produkt in einer Menge von 15,5 g (= 50 % der Theorie) in Form farbloser Kristalle erhalten, Die NMR-Spektren zeigten folgende charakteristische Absorptionen: ¹⁹F-NMR: -68,5 und -81,6 ppm. ¹H-NMR 4,81 ppm.

### Beispiel 12

### 2-(1,1,1,4,4,4-Hexafluor-2-butenoxy)-methoxybenzol

260 g 2-Methoxyphenol wurden in 1 1 Dimethylformamid (technische Qualität) gelöst und mit 220 g 45 %iger Natronlauge versetzt. Dann wurden bei 22°C unter Rühren 400 g 2-Chlor-1,1,1,4,4,4-hexafluor-2-buten zugetropft. Man rührte 2 Stunden bei 22°C nach. Dann versetzte man mit 1,5 1 Eiswasser und extrahierte mit Methylenchlorid.

Die vereinigten organischen Phasen wurden 2 mal mit 10 %iger Natronlauge und 1 mal mit gesättiger NaCl-Lösung gewaschen, mit MgSO₄ getrocknet und destilliert, Die Ausbeute betrug 329 g (58 % der Theorie), der Siedepunkt 68-70°C bei 12 mbar. Die NMR-Spektren zeigten folgende charakteristische Absorptionen: ¹⁹F-NMR: -57,6 und -67,9 ppm. ¹H-NMR: 5,92 ppm.

### Beispiel 13

### 2-(1,1,1,4,4,4-Hexafluor-2-butenoxy)-phenol

286,1 g 2-(1,1,1,4,4,4-Hexafluor-2-butenoxy)-methoxybenzol aus Beispiel 12 wurden in einer Mischung aus 500 ml Eisessig und 500 ml 48 %iger Bromwasserstoffsäure gelöst und mit 5 g Triethylbenzylammoniumchlorid versetzt. Die Mischung wurde bei 150°C Badtemperatur gerührt bis gemäß gaschromatografischer Kontrolle ein vollständiger Umsatz erreicht war. Dann ließ man abkühlen und versetzte mit 2 kg Eiswasser. Die wäßrige Phase wurde mit CH₂Cl₂ gründlich extrahiert. Nach Trocknen mit MgSO₄ wurde das Lösungsmittel abgezogen und der Rückstand im Vakuum destilliert. Die Ausbeute betrug 200 g (50 % der Theorie), der Siedepunkt 80°C bei 16 mbar. Die NMR-Spektren zeigten folgende charakteristische Absorptionen: ¹⁹F-NMR: -59,6 und -69,6 ppm. ¹H-NMR: 6,1 ppm.

### Beispiel 14

### 2-(2,2,2-Trifluorethyl)-2-trifluormethyl-1,3-benzodioxol

200 g 2-(1,1,1,4,4,4-Hexafluor-2-butenoxy)-phenol aus Beispiel 13 wurden in 400 ml Acetonitril gelöst und mit 5 g Triethylamin versetzt. Die Mischung wurde 4 h bei 70°C gerührt. Dann wurde im Vakuum destilliert. Die Ausbeute betrug 162 g (81 % der Theorie), der Siedepunkt 60°C bei 10 mbar. Die NMR-Spektren zeigten folgende charakteristische Absorptionen: ¹⁹F-NMR: -59,0 und -84,6 ppm. ¹H-NMR: 3,02 ppm.

### Beispiel 15

### 2-(2-Chlor-1,1,1,4,4,4-hexafluor-2-butenoxy)-1-benzyloxybenzol

20 g 2-Benzyloxyphenol wurden in 100 ml Dimethylformamid gelöst und mit 9 g 45 %iger Natronlauge versetzt. Dann wurde bei Raumtemperatur 23 g 2,3-Dichlor-1,1,1,4,4,4-hexafluor-2-buten zugetropft. Nach Abklingen der exothermen Reaktion wurde 1 Stunde bei Raumtemperatur nachgerührt, auf Wasser gegeben und mit tert.-Butylmethylether extrahiert. Nach Trocknen mit MgSO₄ wurde das Lösungsmittel abgezogen. Die Ausbeute betrug 29 g (74 % der Theorie). Das ¹⁹F-NMR Spektrum zeigte folgende charakteristische Absorptionen: ¹⁹F-NMR: -59,5; -60,5; -61,7 und -62,8 ppm.

### Beispiel 16

### 2-(2-Chlor-1,1,1,4,4,4-hexafluor-2-butenoxy)-phenol

24,4 g 2-(2-Chlor-1,1,1,4,4,4-hexafluor-2-butenoxy)-1-benzyloxybenzol aus Beispiel 15 wurden in 150 ml Tetrahydrofuran gelöst und bei Raumtemperatur 4 Stunden mit 3 bar Wasserstoff in Gegenwart von 2 g Pd/C (10 %ig) behandelt. Anschließend wurde filtriert, eingeengt und im Vakuum destilliert. Die Ausbeute betrug 13,2 g (69 % der Theorie), der Siedepunkt 56°C bei 0,15 mbar.

### Beispiel 17

### 2-(1-Chlor-2,2,2-trifluorethyl)-2-trifluormethyl-1,3-benzodioxol

11,7 g 2-(2-Chlor-1,1,1,4,4,4-hexafluor-2-butenoxy)-phenol aus Beispiel 16 wurden in 40 ml tert.-Butylmethylether gelöst und mit 40 ml 1n-Natronlauge versetzt. Nach 30 minütigem Rühren bei Raumtemperatur wurde die organische Phase abgetrennt, mit MgSO₄ getrocknet und destilliert. Die Ausbeute betrug 10 g (88 % der Theorie), der Siedepunkt 63°C bei 12 mbar. Die NMR-Spektren zeigten folgende charakteristische Absorptionen: ¹⁹F-NMR: -66,8 und -79,7 ppm. ¹H-NMR: 4,71 ppm.

### Beispiel 18

### 2,2-Dimethyl-4-(1,1,1,4,4,4-hexafluor-2-butenoxy)-1,3-benzodioxol (Formel V, R⁵ gemeinsam mit R¹ = -C(CH₃)₂-O-Rest)

46 g 2,2-Dimethyl-4-hydroxy-1,3-benzodioxol (Formel IV, R⁵ gemeinsam mit R³ = -C(CH₃)₂-O-Rest) wurden in 200 ml N-Methylpyrrolidon gelöst und mit 31 g 40 gew.-%iger wäßriger Natronlauge versetzt. Dann wurde unter Rühren bei Raumtemperatur 54,8 g 2-Chlor-1,1,1,4,4,4-hexafluor-2-buten tropfenweise hinzugefügt. Nach 1 Stunde Nachrühren wurde der Ansatz auf Wasser gegossen und mit tert.-Butylmethylether extrahiert. Die organische Phase wurde mit 10 gew.-%iger wäßriger Natronlauge gewaschen, mit Magnesiumsulfat getrocknet und am Rotationsverdampfer die leicht flüchtigen Anteile entfernt. Es hinterblieben 73,8 g (= 80 % der Theorie) eines gemäß Gaschromatographie 95 % reinen Produktes. Die charakteristischen Absorptionen in den NMR-Spektren waren: ¹⁹F-NMR: -58,1 und -68,5 ppm. ¹H-NMR: 6,73, 6,55, 6,03 und 1,70 ppm.

### Beispiel 19

### 1,2-Dihydroxy-3-(1,1,1,4,4,4-hexafluor-2-butenoxy)-benzol

65 g des Produktes aus Beispiel 18 wurden mit 200 ml konzentrierter wäßriger Salzsäure 4 Stunden lang unter Rühren zum Sieden am Rückfluß erhitzt. Anschließend wurde der Ansatz mit 300 ml Wasser verdünnt und mit Methylenchlorid extrahiert. Nach dem Trocknen mit Magnesiumsulfat wurde aus der organischen Phase das Lösungsmittel abgezogen und 54 g eines zu 90 % reinen Produktes erhalten. Die Umkristallisation aus Cyclohexan ergab farblose Kristalle mit einem Schmelzpunkt von 105°C. Die charakteristischen Absorptionen in den NMR-Spektren waren wie folgt: ¹⁹F-NMR -57,7 und -67,7 ppm. ¹H-NMR: 6,77, 6,50, 6,21 und 5,42 ppm.

### Beispiel 20

### 2-(2,2,2-Trifluorethyl)-2-(trifluormethyl)-4-hydroxy-1,3-benzodioxol (Formel (I), R¹ = OH, X = H, A = CH, R² und R3 = H).

43,5 g des Produkts aus Beispiel 19 wurden in 300 ml Acetonitril gelöst und bei Raumtemperatur mit 1,5 g Triethylamin versetzt. Nach 2 Stunden Rühren bei Raumtemperatur wurde das Lösungsmittel abgezogen und der Rückstand im Vakuum destilliert. Die Ausbeute betrug 17 g (= 39 % der Theorie), der Siedepunkt 85°C bei 0,15 mbar, der Schmelzpunkt 65°C. Die charakteristischen Absorptionen in den NMR-Spektren waren wie folgt:
¹⁹F-NMR: -59,0 und -84,5 ppm.
¹H-NMR: 6,80, 6,55, 6,2 und 3,01 ppm.

### Beispiel 21

### 2,2-Dimethyl-4-(3-chlor-1,1,1,4,4,4-hexafluor-2-butenoxy)-1,3-benzodioxol (Formel (V), R¹ und R⁵ gemeinsam -C(CH₃)₂-O-, X¹ Cl, R² + R³ = H, A = CH).

33,2 g 2,2-Dimethyl-4-hydroxy-1,3-benzodioxol wurden analog Beispiel 18 mit 47 g 2,3-Dichlor-1,1,1,4,4,4-hexafluor-2-buten umgesetzt. Das erhaltene Produkt wurde im Vakuum destilliert und ein 1:1 molares Gemisch aus cis/trans-Isomeren erhalten. Die Ausbeute betrug 51 g (= 70 % der Theorie), der Siedepunkt 70°C bei 0,15 mbar. Die charakteristischen Absorptionen in den NMR-Spektren waren wie folgt; ¹⁹F-NMR: -60,0, -61,6, -62,2 und -63,4 ppm.
¹H-NMR: 6,79, 6,65 bis 6,48 und 1,7 ppm.

### Beispiel 22

### 1,2-Dihydroxy-3-(3-chlor-1,1,1,4,4,4-hexafluor-2-butenoxy)-benzol (Formel (V), R¹ = OH, R² + R³ = H, A = CH, R⁵ = H, X¹ = Cl)

18 g des Produkts aus Beispiel 21 wurden analog Beispiel 19 mit 50 ml konzentrierter Salzsäure umgesetzt. Es wurden 15,7 g eines zu 97 % reinen Produktes erhalten. Das Produkt war ein 1:1 molares Gemisch der cis/trans-Isomere. Die charakteristischen Absorptionen in den NMR-Spektren waren wie folgt:
¹⁹F-NMR: -60,2, -61,3, -62,2 und -63,3 ppm.
¹H-NMR: 6,80, 6,45 und 6,25 ppm.

### Beispiel 23

### 2-(1-Chlor-2,2,2-trifluorethyl)-2-trifluormethyl-4-hydroxy-1,3-benzodioxol

15 g des Produkts aus Beispiel 22 wurden in 50 ml Acetonitril gelöst und mit 1 ml Triethylamin versetzt. Nach 15 minütigem Rühren wurde das Lösungsmittel abgezogen und der Rückstand im Vakuum destilliert. Zur Reinigung wurde das Produkt mit Diethylether aufgenommen und über Siliciumdioxid filtriert. Nach dem Abziehen des Diethylethers verblieben 10,5 g des Produkts (= 70 % der Theorie). Der Schmelzpunkt betrug 139 bis 141°C. Die charakteristischen Absorptionen in den NMR-Spektren waren wie folgt:
¹⁹F-NMR: -66,6 und -79,3 ppm.
¹H-NMR: 8,4, 6,76, 6,60, 6,50 und 4,70 ppm.

### Beispiel 24

### 5-Brom-2-(2,2,2-trifluorethyl)-2-trifluormethyl-1,3-benzodioxol

54,4 g 2-(2,2,2-Trifluorethyl)-2-trifluormethyl-1,3-benzodioxol wurden in 300 ml Tetrachlorkohlenstoff gelöst und mit 0,4 g wasserfreiem Eisen(III)-chlorid versetzt. Dann wurden bei Rückflußtemperatur 32 g Brom zugetropft und bis zum vollständigen Umsatz nachgerührt (Kontrolle durch Gaschromatographie). Dann wurde der Ansatz abkühlen gelassen, mit 10 gew.-%iger wäßriger Natriumhydrogensulfit-Lösung und Wasser gewaschen, mit Magnesiumsulfat getrocknet und eingeengt. Der Rückstand wurde im Vakuum destilliert. Die Ausbeute betrug 58 g (83 % der Theorie), der Siedepunkt 80°C bei 14 mbar. Die charakteristischen Absorptionen in den NMR-Spektren waren wie folgt: ¹⁹F-NMR: -59,2 und -84,9 ppm. ¹H-NMR: 3,02 ppm.

### Beispiel 25

### 5-Brom-2-(1-chlor-2,2,2-trifluorethyl)-2-trifluormethyl-1,3-benzodioxol

51 g 2-(1-Chlor-2,2,2-trifluorethyl)-2-trifluormethyl-1,3-benzodioxol aus Beispiel 2 bzw. 17 wurden in 300 ml Tetrachlorkohlenstoff gelöst und mit 0,5 g wasserfreiem FeCl₃ versetzt. Dann wurden 32 g Brom zugegeben und 3 Stunden unter Rückfluß gerührt. Nach dem Abkühlen wurde mit 10 %iger NaHSO₃-Lösung gewaschen, mit MgSO₄ getrocknet und destilliert. Die Ausbeute betrug 49 g (63 % der Theorie), der Siedepunkt 94-98°C bei 8 mbar.

### Beispiel 26

### 5-Chlor-6-formyl-2-(1-chlor-2,2,2-trifluorethyl)-2-trifluormethyl-1,3-benzodioxol

67 g des Aldehyds aus Beispiel 5 wurden in 150 ml Chloroform gelöst. Bei 50-60°C wurde Chlorgas eingeleitet, bis alles umgesetzt war. Die Rohausbeute nach Abziehen des Lösungsmittels betrug 73 g (98 % der Theorie). Die NMR-Spektren zeigten folgende charakteristische Absorptionen: ¹⁹F-NMR: -68,6 und -81,4 ppm. ¹H-NMR: 4,81 ppm.

### Beispiel 27

### 5-Nitro-2-(2,2,2-trifluorethyl)-2-trifluormethyl-1,3-benzodioxol

Eine Lösung von 54,4 g 2-(2,2,2-Trifluorethyl)-2-trifluormethyl-1,3-benzodioxol in 75 ml Methylenchlorid wurde bei 10°C zu einer Mischung aus 40 ml 65 gew.-%iger Salpetersäure und 40 ml konzentrierter Schwefelsäure getropft. Der Ansatz wurde 1 Stunde bei Raumtemperatur nachgerührt, dann auf Eiswasser gegossen, die organische Phase abgetrennt und die wäßrige Phase mit Methylenchlorid extrahiert. Die vereinigten organischen Phasen wurden mit Wasser gewaschen, getrocknet und von leichtflüchtigen Bestandteilen befreit. Es hinterblieben 95 g des Produkts (= 86 % der Theorie) mit einem Schmelzpunkt von 87 bis 88°C.

Die NMR-Spektren zeigten folgende charakteristischen Absorptionen:
¹⁹F-NMR: -59,0 und -69,4 ppm. ¹H-NMR: 3,10 ppm.

### Beispiel 28

### 5-Nitro-2-(1-chlor-2,2,2-trifluorethyl)-2-trifluormethyl-1,3-benzodioxol

613 g 2-(1-Chlor-2,2,2-trifluorethyl)-2-trifluorme-thyl-1,3-benzodioxol aus Beispiel 2 wurden in 1,2 1 Methylenchlorid gelöst und bei 0-10°C zu einer Mischung aus 400 ml 65 %iger Salpetersäure und 400 ml konz. Schwefelsäure getropft. Man rührte 2 Stunden bei Raumtemperatur nach. Dann wurde vorsichtig auf 2 l Eiswasser gegeben und mit Methylenchlorid extrahiert. Die vereinigten organischen Phasen wurden 2 mal mit Wasser gewaschen, getrocknet und eingeengt. Die Ausbeute betrug 652 g (93 % der Theorie). Die NMR-Spektren zeigten folgende charakteristische Absorptionen: ¹⁹F-NMR: -66,4 und -79,2 ppm. ¹H-NMR: 4,81 ppm.

### Beispiel 29

### 5,6-Dinitro-2-(2,2,2-trifluorethyl)-2-trifluormethyl-1,3-benzodioxol

317 g des Produktes aus Beispiel 27 wurden vorgelegt und dazu unter Rühren eine Mischung aus 250 ml 100 gew.-%iger Salpetersäure und 350 ml konzentrierter Schwefelsäure zugetropft. Die Mischung wurde 2 Stunden bei 55°C gerührt. Dann ließ man den Ansatz abkühlen und goß ihn auf Eiswasser. Das Produkt wurde mit Methylenchlorid extrahiert, mit Natriumhydrogencarbonatlösung neutral gewaschen, getrocknet und am Rotationsverdampfer von leicht flüchtigen Bestandteilen befreit. Die Ausbeute betrug 339 g (= 94 % der Theorie), der Schmelzpunkt 101 bis 103°C.

Die NMR-Spektren zeigten folgende charakteristische Absorptionen:
¹⁹F-NMR: -60,9 und -86,5 ppm. ¹H-NMR: 3,18 ppm.

### Beispiel 30

### 5,6-Dinitro-2-(1-Chlor-2,2,2-trifluorethyl)-2-trifluormethyl-1,3-benzodioxol

352 g 5-Nitro-2-(1-chlor-2,2,2-trifluorethyl)-2-trifluormethyl-1,3-benzodioxol aus Beispiel 28 wurden vorgelegt und mit einer Mischung aus 250 ml 100 gew.-%iger Salpetersäure und 350 ml konzentrierter Schwefelsäure versetzt. Die Mischung wurde 2 Stunden bei 60°C gerührt. Nach dem Abkühlen goß man auf Eiswasser und extrahierte mit Methylenchlorid. Nach Waschen mit Natriumhydrogencarbonatlösung und Trocknen wurde einrotiert. Die Ausbeute betrug 392 g (91 % der Theorie), der Schmelzpunkt 125°C. Die NMR-Spektren zeigten folgende charakteristische Absorptionen; ¹⁹F-NMR: -68,5 und -81,0 ppm. ¹H-NMR: 4,86 ppm.

### Beispiel 31

### 5-Chlorsulfonyl-2-(2,2,2-trifluorethyl)-2-trifluormethyl-1,3-benzodioxol

136 g 2-(2,2,2-Trifluorethyl)-2-trifluormethyl-1,3-benzodioxol wurden in 125 ml Chloroform gelöst. Bei 0°C wurde unter Rühren 175 g Chlorsulfonsäure zugetropft und bei Raumtemperatur bis zum Ende der Gasentwicklung nachgerührt. Dann wurde der Ansatz auf 750 g Eiswasser gegossen, die Phasen getrennt und die wäßrige Phase mit Chloroform extrahiert. Die vereinigten organischen Phasen wurden mit Eiswasser und Natriumhydrogencarbonatlösung gewaschen, mit Magnesiumsulfat getrocknet und am Rotationsverdampfer von leicht flüchtigen Bestandteilen befreit. Es wurden 133 g Produkt erhalten (= 72 % der Theorie), der Schmelzpunkt lag bei 55 bis 57°C. ¹⁹F-NMR: -60,8 und -86,5 ppm. ¹H-NMR: 3,13 ppm.

### Beispiel 32

### 5-Cyano-2-(2,2,2-trifluorethyl)-2-trifluormethyl-1,3-benzodioxol 1

35 g des Produkts aus Beispiel 24 wurden in 75 ml Dimethylformamid gelöst und mit 10,5 g Kupfer(I)cyanid versetzt. Die Mischung wurde 8 Stunden bei 160°C gerührt. Anschließend wurde die heiße Mischung auf 100 ml Eiswasser geschüttet und mit 30 g 1,2-Diaminoethan versetzt. Nach 30 minütigem Rühren wurde die organische Phase abgetrennt und die wäßrige Phase mit Methylenchlorid extrahiert. Die vereinigten organischen Phasen wurden mit einer Lösung von 30 g 1,2-Diaminoethan in 75 ml Wasser gewaschen, mit Magnesiumsulfat getrocknet und im Hochvakuum destilliert. Es wurden 20,5 g des Produktes erhalten (= 69 % der Theorie), Siedepunkt 110°C bei 0,02 mbar. ¹⁹F-NMR: -58,1 und -84,6 ppm. ¹H-NMR: 3,08 ppm.

### Beispiel 33

### 5-Amino-2-(2,2,2-trifluorethyl)-2-trifluormethyl-1,3-benzodioxol

57,4 g des Produkts aus Beispiel 27 wurden in 400 ml Tetrahydrofuran gelöst und in Gegenwart von 4 g Katalysator (Palladium auf Kohle, 10 gew.-%ig) 5 Stunden bei 30°C bei 50 bar mit Wasserstoff hydriert. Danach wurde abfiltriert, das Lösungsmittel entfernt und im Hochvakuum destilliert. Es wurden 37 g Produkt (= 63 % der Theorie) mit einem Siedepunkt von 83°C bei 0,07 mbar erhalten. ¹⁹F-NMR: -59,0 und -84,6 ppm. ¹H-NMR: 2,98 ppm.

### Beispiel 34

### 4-Amino-2-(1-Chlor-2,2,2-trifluorethyl)-2-trifluormethyl-1,3-benzodioxol

84 g 4-Nitro-2-(1-Chlor-2,2,2-trifluorethyl)-2-trifluormethyl-1,3-benzodioxol aus Beispiel 7 wurden in 500 ml Tetrahydrofuran gelöst und an 5 g Palladium auf Kohle (5 %ig) 5 Stunden bei Raumtemperatur mit 15-20 bar Wasserstoff hydriert. Anschließend wurde filtriert, eingeengt und im Vakuum destilliert. Die Ausbeute betrug 31 g (40 % der Theorie), der Siedepunkt 70°C bei 0,1 mbar. Die NMR-Spektren zeigten folgende charakteristische Absorptionen: ¹⁹F-NMR: -68,6 und -81,5 ppm. ¹H-MMR: 4,69 ppm.

### Beispiel 35

### 5-Amino-2-(1-chlor-2,2,2-trifluorethyl)-2-trifluormethyl-1,3-benzodioxol

72 g 5-Nitro-2-(1-chlor-2,2,2-trifluorethyl)-2-trifluormethyl-1,3-benzodioxol aus Beispiel 28 wurden in 500 ml Tetrahydrofuran gelöst und an 5 g Palladium auf Kohle (5 %ig) 5 Stunden bei Raumtemperatur mit 15-20 bar Wasserstoff hydriert. Anschließend wurde filtriert und das Lösungsmittel im Vakuum abgezogen. Die Ausbeute betrug 60 g (93 % der Theorie), der Siedepunkt 80-82°C bei 0,1 mbar. Die NMR-Spektren zeigten folgende charakteristische Absorptionen: ¹⁹F-NMR: -66,5 und -79,4 ppm. ¹H-NMR: 4,68 ppm.

### Beispiel 36

### 5-Isocyanato-2-(2,2,2-trifluorethyl)-2-trifluormethyl-1,3-benzodioxol

37 g des Produkts aus Beispiel 33 wurden in 50 ml absolutem 1,4-Dioxan gelöst und mit einer Lösung von 13,5 g Diphosgen in 80 ml absolutem 1,4-Dioxan versetzt. Dann wurde 6 Stunden bei 110°C (Badtemperatur) gerührt, dann das Lösungsmittel im Vakuum entfernt und der verbleibende Rückstand im Vakuum fraktioniert destilliert. Es wurden 18 g Produkt (= 44 % der Theorie) mit einem Siedepunkt von 63°C bei 0,1 mbar erhalten. ¹⁹F-NMR: -59,3 und -85,0 ppm. ¹H-NMR: 3,0 ppm.

### Beispiel 37

### 5,6-Diamino-2-(2,2,2-trifluorethyl)-2-trifluormethyl-1,3-benzodioxol

339 g des Produkts aus Beispiel 29 wurden in 2000 ml Tetrahydrofuran gelöst und mit 20 g Katalysator (Palladium auf Kohle, 5 gew.-%ig) versetzt. Bei 25 bis 30 bar wurde 13 Stunden lang bei Raumtemperatur mit Wasserstoff hydriert. Anschließend wurde der Ansatz abfiltriert und das Lösungsmittel im Vakuum abgezogen. Es blieb ein Feststoff zurück. Die Ausbeute betrug 274 g (= 96 % der Theorie). ¹⁹F-NMR: -61,2 und -86,6 ppm. ¹H-NMR: 3,02 ppm.

### Beispiel 38

### 2-(1-Chlor-2,2,2-trifluorethyl)-2-(trifluormethyl)-5-hydroxy-1,3-benzodioxol

50 g 2-(1-Chlor-2,2,2-trifluorethyl)-2-(trifluormethyl)-5-formyl-1,3-benzodioxol wurden in 500 ml Methylenchlorid gelöst und mit 53 g 70 gew.-%iger m-Chlorperbenzoesäure versetzt. Der Ansatz wurde 6 Stunden lang unter Rückfluß gerührt. Dann wurde abgekühlt und der ausgefallene Niederschlag abfiltriert. Das Filtrat wurde mit 5 gew.-%iger wäßriger Natriumhydrogensulfit-Lösung und mit gesättigter wäßriger Natriumhydrogencarbonat-Lösung gewaschen und am Rotationsverdampfer eingeengt. Der verbleibende Rückstand wurde in 300 ml Diethylether gelöst und bei Raumtemperatur mit 100 ml In Natronlauge versetzt. Nach beendetem Umsatz wurden die Phasen getrennt und die organische Phase mit gesättigter wäßriger Ammoniumchloridlösung gewaschen, mit Magnesiumsulfat getrocknet und im Vakuum fraktioniert destilliert. Die Ausbeute betrug 26 g (= 54 % der Theorie), der Siedepunkt 95°C bei 0,07 mbar. ¹⁹F-NMR: -68,6 und -81,6 ppm. ¹H-NMR: 4,70 ppm.

### Beispiel 39

### 4-Brommethyl-2-(1-chlor-2,2,2-trifluorethyl)-2-trifluormethyl-1,3-benzodioxol

64 g 4-Methyl-2-(1-chlor-2,2,2-trifluorethyl)-2-trifluormethyl-1,3-benzodioxol aus Beispiel 9 wurden in 500 ml Tetrachlorkohlenstoff gelöst und mit 36 g N-Bromsuccinimid und 0,5 g AIBN (Azoisobuttersäurenitril) versetzt. Die Mischung wurde 3 Stunden unter Rückfluß gerührt, dann abgekühlt und filtriert. Das Lösungsmittel wurde abgezogen und der Rückstand im Vakuum destilliert. Die Ausbeute betrug 57 g (71 % der Theorie), der Siedepunkt 80-82°C bei 0,1 mbar. Das ¹H-NMR-Spektrum zeigte folgende charakteristische Absorption: 4,72 ppm.

### Beispiel 40

### 2-(2,2,2-Trifluorethyl)-2-trifluormethyl-1,3-benzodioxol

306,5 g 2-(1-Chlor-2,2,2-trifluorethyl)-2-trifluormethyl-1,3-benzodioxol aus Beispiel 2 wurden in 500 ml THF gelöst und mit 101 g Triethylamin und 30 g Palladium auf Kohle (5 gew.-%ig) versetzt. Dann wurde 48 h bei 110°C mit 100 bar Wasserstoff hydriert. Anschließend wurde filtriert, das Lösungsmittel abgezogen und der Rückstand im Vakuum fraktioniert. Die Ausbeute betrug 126 g (46 % der Theorie), der Siedepunkt 60°C bei 10 mbar. Die NMR-Spektren zeigten folgende charakteristische Absorptionen: ¹⁹F-NMR: -59,0 und -84,6 ppm. ¹H-NMR; 3,02 ppm.

## Patentansprüche

1. Fluorierte 1,3-Benzo- und 1,3-Pyridodioxole der Formel (I) 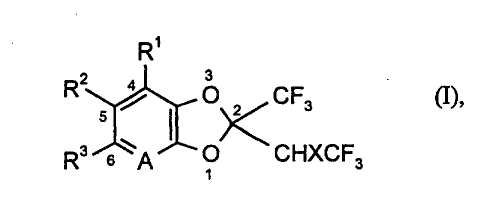 in der
A für C-R⁴ oder N und
X für Wasserstoff, Fluor, Chlor oder Brom stehen und
R¹ bis R⁴ gleich oder verschieden voneinander sein können und jeweils Wasserstoff, Halogen, C₁-C₆-Alkyl, C₁-C₆-Alkoxy, Halogen-C₁-C₆-alkyl, C₆-C₁₀-Aryl, CHO, COOH, COCl, CN, OH, NCO, COO-C₁-C₆-Alkyl, NO₂, NH₂, NH-C₁-C₆-Alkyl, N(C₁-C₆-Alkyl)₂, SO₂Cl, SO₃H, SO₃Na oder SO₃K bedeuten,

2. Fluorierte 1,3-Benzo- und 1,3-Pyridodioxole nach Anspruch 1, **dadurch gekennzeichnet, daß** in Formel (I) mindestens zwei der Reste R¹ bis R⁴ für Wasserstoff und der oder die verbleibenden Reste, soweit sie nicht für Wasserstoff stehen, unabhängig voneinander für Chlor, Brom, Methyl, Ethyl, CH₂Cl, CH₂Br, Phenyl, CHO, COOH, OH, NCO, NO₂, NH₂ und/oder SO₂Cl stehen.

3. Verfahren zur Herstellung von 1,3-Benzo- und 1,3-Pyridodioxolen des Anspruchs 1, **dadurch gekennzeichnet, daß** man 1,2-Dihydroxybenzole oder 2,3-Dihydroxypyridine der Formel (II) 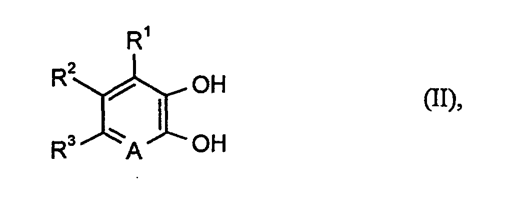 in der
A und R¹ bis R³ die in Anspruch 1 angegebene Bedeutung haben, R¹ bis R³ jedoch nicht für OH, COCl oder SO₂Cl stehen,
in Gegenwart einer Base und eines Verdünnungsmittels bei -20 bis +200°C mit einem Hexafluorbuten der Formel (III) umsetzt 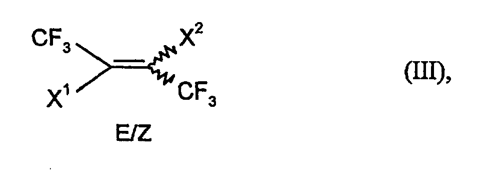 in der
X¹ für Wasserstoff oder Halogen und
X² für Halogen stehen.

4. Verfahren nach Anspruch 3, **dadurch gekennzeichnet, daß** man auf 1 Mol Dihydroxyverbindung der Formel (II) 0,8 bis 1,2 Mole Hexafluorbuten der Formel (III) und als Basen 1 bis 5 Äquivalente pro Mol der Verbindung der Formel (II) von Natriumhydroxid, Kaliumhydroxid, Natriumcarbonat, Kaliumcarbonat, DBN, DBU, Pyridin und/oder aliphatischen Aminen einsetzt.

5. Verfahren zur Herstellung von 1,3-Benzo- und 1,3-Pyridodioxolen des Anspruchs 1, **dadurch gekennzeichnet, daß** man mit einer Schutzgruppe versehene 1,2-Dihydroxybenzole oder 2,3-Dihydroxypyridine der Formel (IV) 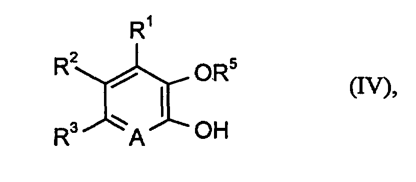 in der
A und R¹ bis R³ die in Anspruch 1 angegebene Bedeutung haben und
R⁵ für eine Schutzgruppe oder
R⁵ gemeinsam mit R¹ für einen -C(CH₃)₂-O-Rest stehen,
zunächst mit einem Hexafluorbuten der Formel (III) umsetzt 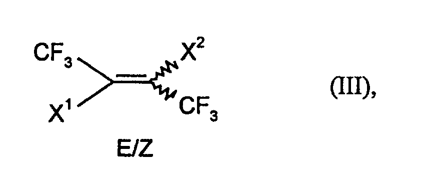 in der
X¹ für Wasserstoff oder Halogen und
X² für Halogen stehen,
so ein Zwischenprodukt der Formel (V) erhält, 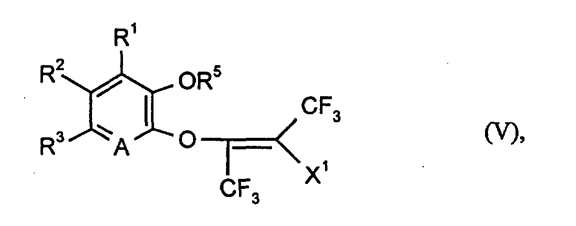 in der
A und R¹ bis R³ die bei Formel (I),
R⁵ die bei Formel (IV) und
X¹ die bei Formel (III)
angegebene Bedeutung haben,
aus dem Zwischenprodukt der Formel (V) die Schutzgruppe R⁵ abspaltet,
die so erhältliche OH-Verbindung mit einer Base umsetzt und so 1,3-Benzo- oder 1,3-Pyrido-dioxole der Formel (I) erhält.

6. Verfahren nach Anspruch 5, **dadurch gekennzeichnet, daß** man es bei -10 bis +50°C und mit einer Basenmenge von 1,0 bis 1,2 Äquivalenten pro Mol der Verbindung der Formel (TV) durchführt.

7. Verfahren zur Modifizierung von Verbindungen des Anspruchs 1, **dadurch gekennzeichnet, daß** man durch elektrophile aromatische Substitution Nitro- oder Halogengruppen einführt, Halogengruppen gegen Cyanogruppen austauscht, Alkylgruppen zu Halogenalkyl-Seitengruppen halogeniert oder NO ₂-Gruppen zu NH₂-Gruppen hydriert.

8. Verfahren zur Modifizierung von Verbindungen des Anspruchs 1, **dadurch gekennzeichnet, daß** man 1,3-Benzo- oder 1,3-Pyridodioxole des Anspruchs 1 mit X = Chlor hydriert.

9. Die Verwendung von 1,3-Benzo- und 1,3-Pyridodioxolen des Anspruchs 1 zur Herstellung von Pflanzenschutzmitteln der Formel 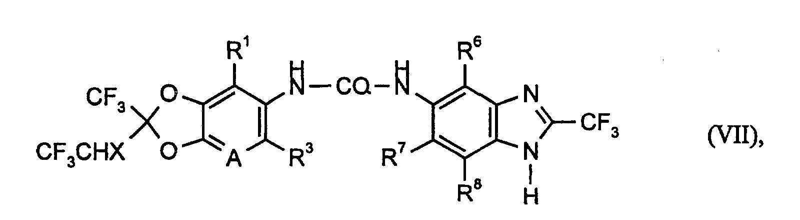 in welcher
R¹, R³, X und A die bei Anspruch 1 angegebene Bedeutung haben und R⁶, R⁷ und R⁸ unabhängig voneinander jeweils für Wasserstoff, Halogen, Cyano, Nitro, für Alkyl, Alkoxy, Alkylthio, Alkylsulfinyl, Alkylsulfonyl oder Cycloalkyl, für ankondensiertes Dioxyalkylen, für Hydroxycarbonyl, Alkylcarbonyl, Alkoxycarbonyl, Cycloalkyloxycarbonyl, für Amino oder Aminocarbonyl oder für Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen, wobei jedoch mindestens einer der Substituenten R⁶, R⁷ oder R⁸ für Halogenalkyl mit Ausnahme des Chlormethylrestes, Halogenalkoxy, Halogenalkylthio, Halogenalkylsulfinyl, Halogenalkylsulfonyl, Alkylsulfonyl, für ankondensiertes Dioxyalkylen, für Hydroxycarbonyl, Alkylcarbonyl, Alkoxycarbonyl, Cycloalkyloxycarbonyl, für Aryl, Arylthio, Arylsulfinyl, Arylsulfonyl, Arylsulfonyloxy, Arylcarbonyl, Aryloxycarbonyl, Arylazo oder Arylthiomethylsulfonyl steht,
oder der Formel 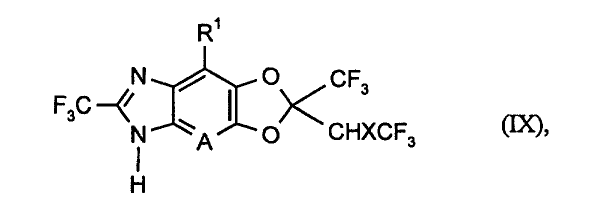 in der
A, R¹ und X die in Anspruch 1 angegebene Bedeutung haben.

## Claims

1. Fluorinated 1,3-benzo- and 1,3-pyrido-dioxoles of formula (I) 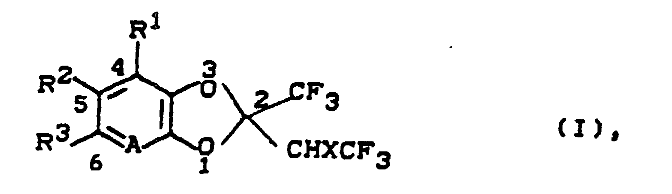 in which
A is C-R⁴ or N,
X is hydrogen, fluorine, chlorine or bromine and
R¹ to R⁴ can be identical to or different from one another and are each hydrogen, halogen, C₁-C₆-alkyl, C₁-C₆-alkoxy, halogeno-C₁-C₆-alkyl, C₆-C₁₀-aryl, CHO, COOH, COCl, CN, OH, NCO, COO-C₁-C₆-alkyl, NO₂, NH₂, NH-C₁-C₆-alkyl, N(C₁-C₆-alkyl)₂, SO₂Cl, SO₃H, SO₃Na or SO₃K.

2. Fluorinated 1,3-benzo- and 1,3-pyrido-dioxoles according to Claim 1, **characterised in that**, in formula (I), at least two of the radicals R¹ to R⁴ are hydrogen and the remaining radical or radicals, provided they are not hydrogen, independently of one another are chlorine, bromine, methyl, ethyl, CH₂Cl, CH₂Br, phenyl, CHO, COOH, OH, NCO, NO₂, NH₂ and/or SO₂Cl.

3. Process for the preparation of 1,3-benzo- and 1,3-pyrido-dioxoles of Claim 1, **characterised in that** 1,2-dihydroxybenzenes or 2,3-dihydroxypyridines of formula (II): 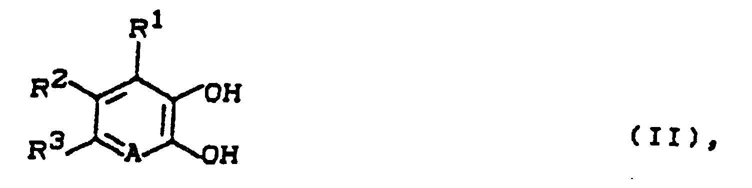 in which
A and R¹ to R³ are as defined in Claim 1 except that R¹ to R³ are not OH, COCl or SO₂Cl,
are reacted, in the presence of a base and a diluent, at -20 to +200°C, with a hexafluorobutene of formula (III): 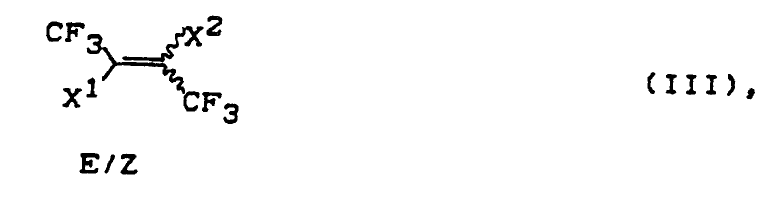 in which
X¹ is hydrogen or halogen and
X² is halogen.

4. Process according to Claim 3, **characterised in that** 0.8 to 1.2 mol of hexafluorobutene of formula (III) are used per mol of dihydroxy compound of formula (II) and, as bases, 1 to 5 equivalents of sodium hydroxide, potassium hydroxide, sodium carbonate, potassium carbonate, DBN, DBU, pyridine and/or aliphatic amines are used per mol of compound of formula (II).

5. Process for the preparation of 1,3-benzo- and 1,3-pyrido-dioxoles of Claim 1, **characterised in that** 1,2-dihydroxybenzenes or 2,3-dihydroxypyridines, carrying a protecting group, of formula (IV) : 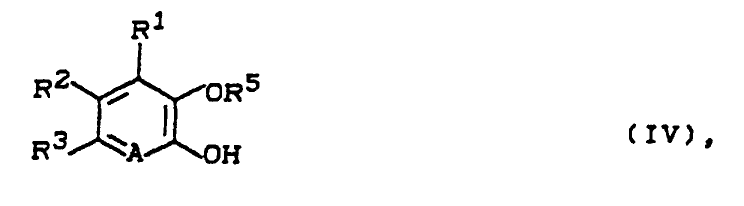 in which
A and R¹ to R³ are as defined in Claim 1 and
R⁵ is a protecting group or
R⁵ and R¹ together are a -C(CH₃)₂-O- radical,
are initially reacted with a hexafluorobutene of formula (III): 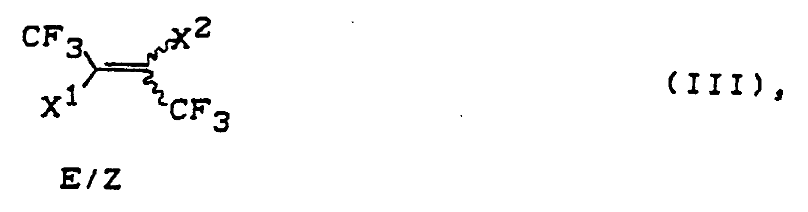 in which
X¹ is hydrogen or halogen and
X² is halogen,
to give an intermediate of formula (V): 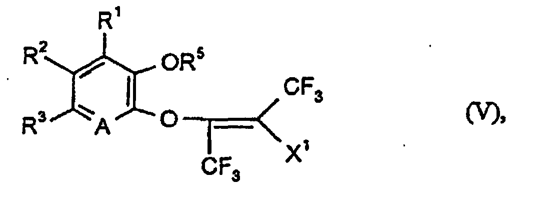 in which
A and R¹ to R³ are as defined for formula (I),
R⁵ is as defined for formula (IV) and
X¹ is as defined for formula (III),
the protecting group R⁵ is cleaved from the intermediate of formula (V) and the resulting OH compound is reacted with a base to give 1,3-benzo- or 1,3-pyrido-dioxoles of formula (I).

6. Process according to Claim 5, **characterised in that** it is carried out at -10 to +50°C and with an amount of 1.0 to 1.2 equivalents of base per mol of compound of formula (IV).

7. Process for the modification of compounds of Claim 1, **characterised in that** nitro or halogen groups are introduced by electrophilic aromatic substitution, halogen groups are exchanged with cyano groups, alkyl groups are halogenated to halogenoalkyl side-groups or NO₂ groups are hydrogenated to NH₂ groups.

8. Process for the modification of compounds of Claim 1, **characterised in that** 1,3-benzo- or 1,3-pyridodioxoles of Claim 1 in which X = chlorine are hydrogenated.

9. Use of 1,3-benzo- and 1,3-pyrido-dioxoles of Claim 1 for the preparation of plant protection agents of the formula 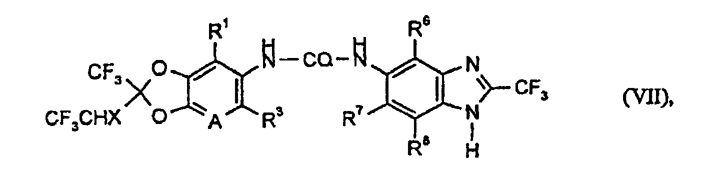 in which
R¹, R³, X and A are as defined in Claim 1 and R⁶, R⁷ and R⁸ independently of one another are each hydrogen, halogen, cyano or nitro, alkyl, alkoxy, alkylthio, alkylsulphinyl, alkylsulphonyl or cycloalkyl, fused dioxyalkylene, hydroxycarbonyl, alkylcarbonyl, alkoxycarbonyl or cycloalkyloxycarbonyl, amino or aminocarbonyl, or aryl, aryloxy, arylthio, arylsulphinyl, arylsulphonyl, arylsulphonyloxy, arylcarbonyl, aryloxycarbonyl, arylazo or arylthiomethylsulphonyl, at least one of the substituents R⁶, R⁷ or R⁸ being halogenoalkyl except for the chloromethyl radical, halogenoalkoxy, halogenoalkylthio, halogenoalkylsulphinyl, halogenoalkylsulphonyl or alkylsulphonyl, fused dioxyalkylene, hydroxycarbonyl, alkylcarbonyl, alkoxycarbonyl or cycloalkyloxycarbonyl, or aryl, arylthio, arylsulphinyl, arylsulphonyl, arylsulphonyloxy, arylcarbonyl, aryloxycarbonyl, arylazo or arylthiomethylsulphonyl,
or of the formula 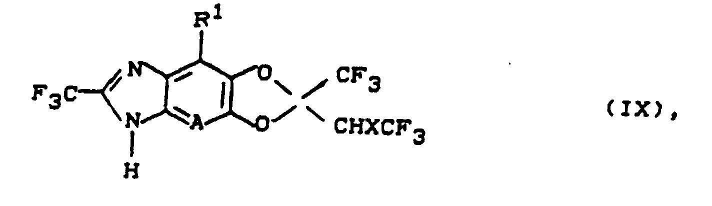 in which
A, R¹ and X are as defined in Claim 1.

## Revendications

1. 1,3-benzo- et 1,3-pyridodioxoles fluorés de formule (I) 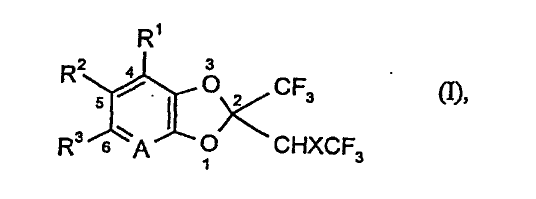 dans laquelle
A représente C-R⁴ ou N et
X est l'hydrogène, le fluor, le chlore ou le brome, et
R¹ à R⁴ peuvent être identiques ou différents les uns des autres et représentent chacun l'hydrogène, un halogène, un groupe alkyle en C₁ à C₆, alkoxy en C₁ à C₆, halogénalkyle en C₁ à C₆, aryle en C₆ à C₁₀, CHO, COOH, COCl, CN, OH, NCO, COO-(alkyle en C₁ à C₆), NO₂, NH₂, NH-(alkyle en C₁ à C₆), N(alkyle en C₁ à C₆)₂, SO₂Cl, SO₃H, SO₃Na ou SO₃K.

2. 1,3-benzo- et 1,3-pyridodioxoles fluorés suivant la revendication 1, **caractérisés en ce que**, dans la formule (I), au moins deux des restes R¹ à R⁴ représentent l'hydrogène et le ou les autres restes, dans la mesure où ils ne sont pas l'hydrogène, représentent, indépendamment les uns des autres, le chlore, le brome, un groupe méthyle, éthyle, CH₂Cl, CH₂Br, phényle, CHO, COOH, OH, NCO, NO₂, NH₂ et/ou SO₂Cl.

3. Procédé de production de 1,3-benzo- et 1,3-pyridodioxoles suivant la revendication 1, **caractérisé en ce qu'**on fait réagir des 1,2-dihydroxybenzènes ou des 2,3-dihydroxypyridines de formule (II) 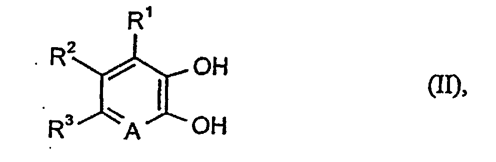 dans laquelle
A et R¹ à R³ ont la définition indiquée dans la revendication 1, R¹ à R³ ne représentant toutefois pas un groupe OH, COCl ou SO₂Cl,
en présence d'une base et d'un diluant à une température de -20 à +200°C avec un hexafluorobutène de formule (III) 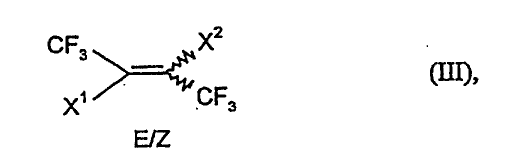 dans laquelle
X¹ est l'hydrogène ou un halogène et
X² est un halogène.

4. Procédé suivant la revendication 3, **caractérisé en ce qu'**on utilise pour 1 mole de composé dihydroxylé de formule (II) 0,8 à 1,2 mole d'hexafluorobutène de formule (III) et comme bases, 1 à 5 équivalents, par mole du composé de formule (II), d'hydroxyde de sodium, d'hydroxyde de potassium, de carbonate de sodium, de carbonate de potassium, de DBN, de DBU, de pyridine et/ou d'amines aliphatiques.

5. Procédé de production de 1,3-benzo- et 1,3-pyridodioxoles suivant la revendication 1, **caractérisé en ce qu'**on fait réagir des 1,2-dihydroxybenzènes ou des 2,3-dihydroxypyridines pourvus d'un groupe protecteur de formule (IV) 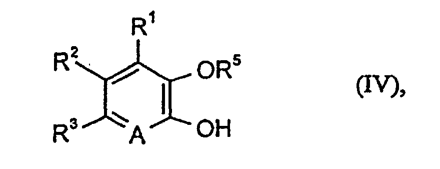 dans laquelle
A et R¹ à R³ ont la définition indiquée dans la revendication 1 et
R⁵ représente un groupe protecteur, ou bien
R⁵ forme conjointement avec R¹ un reste -C(CH₃)₂-O-,
tout d'abord avec un hexafluorobutène de formule (III) 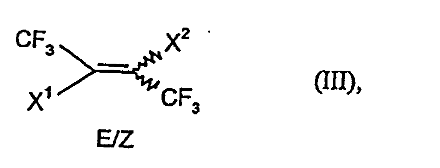 dans laquelle
X¹ est l'hydrogène ou un halogène et
X² est un halogène,
on obtient ainsi un produit intermédiaire de formule (V) 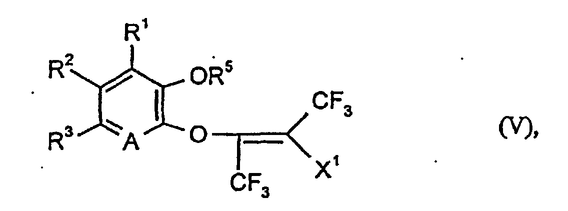 dans laquelle
A et R¹ à R³ ont la définition indiquée pour la formule (I),
R⁵ a la définition indiquée pour la formule (IV) et
X¹ a la définition indiquée pour la formulé (III),
on élimine le groupe protecteur R⁵ du produit intermédiaire de formule (V),
on fait réagir le composé hydroxylé ainsi obtenu avec une base et on obtient ainsi des 1,3-benzo- ou 1,3-pyridodioxoles de formule (I).

6. Procédé suivant la revendication 5, **caractérisé en ce qu'**il est mis en oeuvre à une température de -10 à +50°C et avec une quantité de bases de 1,0 à 1,2 équivalent par mole du composé de formule (IV).

7. Procédé de modification de composés suivant la revendication 1, **caractérisé en ce qu'**on introduit des groupes nitro ou halogéno par substitution aromatique électrophile, on remplace des groupes halogéno par des groupes cyano, on halogène des groupes alkyle en groupes latéraux halogénalkyle ou on hydrogène des groupes NO₂ en groupes NH₂.

8. Procédé de modification de composés suivant la revendication 1, **caractérisé en ce qu'**on hydrogène des 1,3-benzo- ou 1,3-pyridodioxoles suivant la revendication 1 dans lesquels X représente le chlore.

9. Utilisation de 1,3-benzo- et 1,3-pyridodioxoles suivant la revendication 1 pour la préparation d'agents phytosanitaires de formule 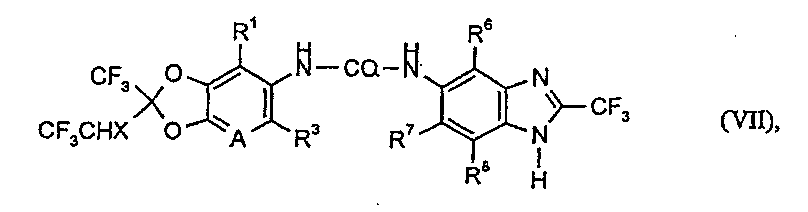 dans laquelle
R¹, R³, X et A ont la définition indiquée dans la revendication 1 et R⁶, R⁷ et R⁸ représentent, indépendamment les uns des autres, l'hydrogène, un halogène, un groupe cyano, nitro, un groupe alkyle, alkoxy, alkylthio, alkylsulfinyle, alkylsulfonyle ou cycloalkyle, un groupe dioxyalkylène condensé, un groupe hydroxycarbonyle, alkylcarbonyle, alkoxycarbonyle, cycloalkyloxycarbonyle, un groupe amino ou aminocarbonyle ou un groupe aryle, aryloxy, arylthio, arylsulfinyle, arylsulfonyle, arylsulfonyloxy, arylcarbonyle, aryloxycarbonyle, arylazo ou arylthiométhylsulfonyle, toutefois l'un au moins des substituants R⁶, R⁷ ou R⁸ représente un reste halogénalkyle à l'exception du reste chlorométhyle, un groupe halogénalkoxy, halogénalkylthio, halogénalkylsulfinyle, halogénalkylsulfonyle, alkylsulfonyle, un groupe dioxyalkylène condensé, un groupe hydroxycarbonyle, alkylcarbonyle, alkoxycarbonyle, cycloalkyloxycarbonyle, un groupe aryle, arylthio, arylsulfinyle, arylsulfonyle, arylsulfonyloxy, arylcarbonyle, aryloxycarbonyle, arylazo ou arylthiométhylsulfonyle,
ou de formule 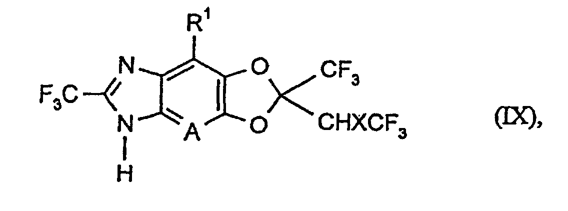 dans laquelle
A, R¹ et X ont la définition indiquée dans la revendication 1.
